# EUROPÄISCHE PATENTSCHRIFT

(11) **EP 2 881 038 B1**
(45) Veröffentlichungstag und Bekanntmachung des Hinweises auf die Patenterteilung: **03.01.2018**
(21) Anmeldenummer: 14195484.2
(22) Anmeldetag: 28.11.2014
(51) Int. Cl.: A61B 5/11, G08B 21/22

(54) **System zum Überwachen von Bewegungen einer Person**
System for monitoring movements of a person
Système de surveillance des mouvements d'une personne

(30) Priorität: 28.11.2013 DE 102013224425
(43) Veröffentlichungstag der Anmeldung: 10.06.2015
(73) Patentinhaber: CubileHealth Gmbh, 6020 Innsbruck (AT)
(72) Erfinder: Hilbe, Johannes, 6020 Innsbruck (AT)
(74) Vertreter: Dilg, Haeusler, Schindelmann Patentanwaltsgesellschaft mbH

(56) Entgegenhaltungen:
- EP-A1- 2 098 206
- US-B1- 8 419 660

## Beschreibung

### Technisches Gebiet

Die vorliegende Erfindung betrifft eine Polstervorrichtung und ein Polstersystem zum Überwachen von Bewegungen einer Person, sowie ein Verfahren zum Überwachen von Bewegungen einer Person.

### Hintergrund der Erfindung

In Krankenhäusern oder Pflegeheimen, wie auch bei ambulanter oder familiärer Pflege, ist es notwendig, die Bewegungen von Patienten, beispielsweise in einem Bett oder in einem Sessel, zu überwachen, um eine Fehlposition eines Patienten früh zu erkennen. Beispielsweise ist es notwendig, einen Patienten im Schlaf zu überwachen, um frühzeitig zu erkennen und gegebenenfalls zu verhindern, dass der Patient aus dem Bett herausfällt.

Da eine Überwachung der Patientenposition durch das Pflegepersonal oder Krankenhauspersonal zeit- und kostenintensiv ist, werden automatische Überwachungssysteme eingesetzt. Automatische Überwachungssysteme überwachen automatisch den Aufenthaltsort bzw. die Bewegung des Patienten und geben ggf. ein Alarmsignal an das Pflegepersonal bzw. Krankenhauspersonal weiter.

Es ist bekannt, Drucksensoren bereitzustellen, welche in Auflageflächen eines Sitzes oder eines Betts eingerichtet sind, um eine Druckbelastung zu detektieren. Bei einer Druckveränderung, z.B. bei einer Druckentlastung, kann beispielsweise eine Änderung der Patientenposition detektiert werden und ggf. Alarm ausgelöst werden.

EP 2 098 206 B1 offenbart ein Alarmsystem, bei welchem beispielsweise in einer Matratze eines Krankenhausbetts Luftschläuche angeordnet sind. Die Luftschläuche sind luftdicht. Ferner ist an dem Luftschlauch ein Drucksensor gekoppelt, um eine Druckdifferenz, beispielsweise aufgrund eines Zusammenquetschens der Schläuche, zu messen. In der US8419660B1 wird eine Polstervorrichtung beschrieben, die der vorliegenden Erfindung ähnelt.

Die bekannten Alarmsysteme benötigen aufwändige Installationen beispielsweise in einer Matratze und sind bereits ab Herstellung auf einen bestimmten Einsatzbereich, beispielsweise als Matratze für ein Krankenhausbett, festgelegt.

### Darstellung der Erfindung

Es ist eine Aufgabe der vorliegenden Erfindung, ein einfach herzustellendes und gleichzeitig flexibel einsetzbares Überwachungssystem für Personen (Patienten) bereitzustellen.
Diese Aufgabe wird durch eine Polstervorrichtung zum Überwachen von Bewegungen einer Person, bei welchem ein Polstermodul mit einem offenzelligen Schaumstoff als Füllmaterial eingesetzt wird, sowie mit einem Verfahren zum Überwachen von Bewegungen einer Person gemäß den unabhängigen Patentansprüchen gelöst.
Gemäß einem ersten Aspekt der vorliegenden Erfindung wird eine Polstervorrichtung zum Überwachen von Bewegungen einer Person beschrieben. Die Polstervorrichtung weist ein Polstermodul auf.
Auf das Polstermodul ist zumindest ein Anteil eines Körpergewichts einer Person (Patienten) übertragbar. Das Polstermodul weist ein Innenvolumen auf, in welchem ein Füllmaterial aus einem offenzelligen Schaumstoff angeordnet ist, wobei der offenzellige Schaumstoff fluiddurchlässige Zellwände aufweist. Das Füllmaterial ist mit einer Umhüllung fluiddicht umhüllt, sodass bei Änderung einer Belastung des Polstermoduls eine Änderung einer physikalischen Größe (Massenstrom) eines Fluides (z.B. flüssig oder gasförmig, insbesondere Luft oder Wasser) innerhalb des Polstermoduls generierbar ist. Ein Koppelelement des Polstermoduls ist mit dem Innenvolumen gekoppelt, wobei an das Koppelelement ein Sensor( Durchflusssensor) derart ankoppelbar ist, dass mittels des Sensors die Änderung der physikalischen Größe des Fluides in dem Innenvolumen messbar ist.
Ein Koppelelement kann beispielsweise ein Ventil, einen Verschluss oder eine Öffnung aufweisen, woran ein entsprechender Sensor direkt oder indirekt mittels eines Verbindungsschlauchs angekoppelt werden kann. Das Koppelelement kann entsprechend zudem ein Innen-/oder Außengewinde aufweisen, um den Sensor anzukoppeln. Ferner kann das Koppelelement ausgebildet sein, um mit dem Sensor oder dem Verbindungsschlauch einen Bajonettverschluss auszubilden. Das Polstermodul weist ferner den Sensor zur Messung der Änderung der physikalischen Größe des Fluides auf, welcher in dem Polstermodul angeordnet ist und mittels des Koppelelements an das Innenvolumen gekoppelt ist. Der Sensor ist mit einer Ausleseeinheit derart koppelbar, dass Signale, welche indikativ für die Änderung der physikalischen Größe des Fluides sind, von der Ausleseeinheit auslesbar sind.
Der Sensor kann direkt im Innenvolumen des Polstermoduls angeordnet sein oder in einem Zwischenbereich zwischen dem Innenvolumen und einer Außenhülle des Polstermoduls. Das Koppelelement kann z.B. eine Fixierung des Sensors im Innenvolumen oder im Zwischenbereich ermöglichen, z.B. mittels einer Klettverbindung. Das Polstermodul weist den Sensor auf, welcher außerhalb des Polstermoduls angeordnet ist und mittels des Koppelelements an das Innenvolumen gekoppelt ist. Der Sensor ist mit einer Ausleseeinheit derart koppelbar, dass Signale, welche indikativ für die Änderung der physikalischen Größe des Fluides sind, von der Ausleseeinheit auslesbar sind.
Wird der Sensor außerhalb der Polstermodule angeordnet, können die Polstermodule ohne elektrische Bauteile/Sensoren hergestellt und vertrieben werden. Die Polstermodule sind dann über den Verbindungsschlauch mit dem Sensor bzw. der Steuereinheit verbunden. Der Sensor als Durchflusssensor kann beispielsweise die Durchflussrate (in beiden Richtungen) messen und somit auf die Belastung des Polstermoduls schließen. Der Sensor als Drucksensor kann beispielsweise die Druckänderung messen und somit auf die Belastung des Polstermoduls schließen.
Der Sensor kann beispielsweise in einem Steuergehäuse bzw. einer Steuereinheit angeordnet werden, welche außerhalb des Polstermoduls angeordnet ist. Die Steuereinheit ist beispielsweise an einem Bett befestigt, auf welchem ein Polstermodul aufliegt. Der Sensor ist mit einem Verbindungsschlauch mit dem Koppelelement (z.B. Ventil) des Polstermoduls (lösbar) angekoppelt, sodass die Änderung der physikalischen Größe des Fluides im Innenvolumen des Polstermoduls von dem Sensor messbar ist. Der Sensor kann insbesondere ausgebildet sein, um einen Absolutwert zu messen, d.h. ohne einen Referenzwert z.B. der Umgebung des Polstermoduls zu beziehen. Der Sensor kann somit in dem Innenvolumen des Polstermoduls ohne Verbindung zu der Umgebung des Polstermoduls angeordnet sein.

Gemäß einem weiteren Aspekt der vorliegenden Erfindung gemäß Anspruch 8 wird ein Verfahren zum Überwachen von Bewegungen einer Person beschrieben. Gemäß dem Verfahren wird eine Änderung der physikalischen Größe des Fluides in einem Innenvolumen eines Polstermoduls mittels eines Sensors, welcher in dem Innenvolumen angeordnet sein kann oder außerhalb des Polstermoduls angeordnet sein kann, gemessen. Der Sensor ist an ein Koppelelement gekoppelt, wobei das Koppelelement mit dem Innenvolumen gekoppelt ist, sodass mittels des Sensors die Änderung der physikalischen Größe des Fluides in dem Innenvolumen messbar ist.
Auf das Polstermodul ist zumindest ein Anteil eines Körpergewichts einer Person übertragbar, wobei in dem Polstermodul ein Füllmaterial aus einem offenzelligen Schaumstoff angeordnet ist. Der offenzellige Schaumstoff weist fluiddurchlässige Zellwände auf, wobei das Füllmaterial mit einer Umhüllung fluiddicht umhüllt ist, sodass bei Änderung einer Belastung des Polstermoduls die Änderung der physikalischen Größe des Fluides innerhalb des Polstermoduls generierbar ist. Gemäß dem Verfahren werden ferner Signale des Sensors ausgelesen, welche indikativ für die Änderung der physikalischen Größe des Fluides sind. Die Ausleseeinheit ist dazu mit dem Sensor gekoppelt. Das Polstermodul bildet beispielsweise eine Matratze, eine Matratzenhülle, ein Sitzkissen, ein Rückenpolster oder eine Bodenauflage aus. Das Polstermodul kann beispielsweise auch unter eine herkömmliche Matratze gelegt werden und z.B. an einem Lattenrost befestigt werden. So können beispielsweise Betten mit den Polstermodulen ausgestatten werden und die Betten anschließend mit herkömmlichen Matratzen bestückt werden. Das Polstermodul ist elastisch verformbar ausgebildet und stellt eine weiche Auflagefläche für einen Körper, bzw. einer Person/Patienten, dar. Der Patient kann sich beispielsweise auf das Polstermodul legen, stellen oder setzen und zumindest einen Anteil seines Körpergewichts oder sein gesamtes Körpergewicht auf das Polstermodul übertragen. Die Polstervorrichtung kann ferner zwei oder mehr Polstermodule aufweisen. Die Polstermodule können beispielsweise gemäß dem unten beschriebenen System mittels Anschlusselementen miteinander gekoppelt werden.

Im Inneren des Polstermoduls ist das Innenvolumen ausgebildet. In dem Innenvolumen kann beispielsweise ein weiches Füllmaterial, wie beispielsweise Federn, Schaumstoff oder ein sonstiger weicher, elastischer Füllstoff eingesetzt werden.

Das Polstermodul kann eine flächige Grundform aufweisen. Ferner kann das Polsterelement als Schlauch ausgebildet sein. Der Schlauch weist beispielsweise einen runden Querschnitt mit einem bestimmten Durchmesser auf, wobei der Schlauch sich entlang einer Längsrichtung erstreckt. Der Schlauch ist flexibel ausgebildet und kann entlang einer gewünschten, z.B. kurvigen, schleifenförmigen oder mäanderförmigen, Bahn verlegt werden. So kann das Polstermodul als Schlauch unter eine Matratze gelegt werden. Der Schlauch kann in einer beliebigen Bahn unter der Matratze verlegt werden und somit insbesondere unter gewünschte Stellen der Matratze gelegt werden, um Druckveränderungen an gewünschten Bereichen der Matratze zu messen.

Das Polstermodul bzw. dessen Innenvolumen kann ferner mit dem Fluid bedruckt werden, so dass die Härte des Polstermoduls einstellbar ist. Das Fluid kann über ein Ventil in das Innenvolumen einströmen oder ausströmen. Ferner kann ein Überdruckventil Druckspitzen im Innenvolumen verhindern und das Fluid bei einem gewissen Grenzdruck nach außen abführen.

Gemäß dem oben beschriebenen Aspekt der vorliegenden Erfindung besteht das Füllmaterial ganz oder bereichsweise aus einem offenzelligen (bzw. offenporigen) Schaumstoff, wobei der offenzellige Schaumstoff fluiddurchlässige Zellwände aufweist. Der offenzellige Schaumstoff ist zusammendrückbar, um mittels der Druckbelastung sein Volumen zu verkleinern. Bei offenzelligen Schaumstoffen sind die Zellwände nicht geschlossen, so dass diese ein Fluid aufnehmen und durchlassen können.

Das Füllmaterial in dem Polstermodul ist mit einer Umhüllung fluiddicht umhüllt. Die Umhüllung kann beispielsweise als fluiddichte Folie ausgebildet sein. Wird das Polstermodul und entsprechend das Innenvolumen bzw. das Füllmaterial durch Belastung der Person zusammengedrückt, so ändert sich die physikalischen Größe (z.B. Druck oder Durchflussmenge durch das Koppelelement) des Fluides im Inneren des Innenvolumens bzw. des Füllmaterials.

Das offenzellige Füllmaterial erlaubt es, über seine gesamte Erstreckung einen fluiddurchlässigen Bereich auszubilden. Mit anderen Worten bilden sich entlang des Füllmaterials keine geschlossenen Zellen aus. Dies führt dazu, dass unabhängig von dem Belastungsort durch der Person am Füllmaterial bzw. am Polstermodul und unabhängig von dem Einbauort des Sensors relativ zu dem Belastungsort, eine Änderung der physikalischen Größe des Fluides (z.B. eine Druckänderung) in dem Belastungsbereich gemessen werden kann. Mit anderen Worten stehen alle Bereiche des Füllmaterials in Wirkverbindung zueinander, so dass die Anordnung eines einzigen Sensors ausreicht, um eine Änderung der physikalischen Größe des Fluides entlang des gesamten Bereichs des Füllmaterials zu messen.

Somit sind beispielsweise extra eingearbeitete Druckmessleitungen oder verteilt angeordnete Sensoren unnötig, so dass eine einfache und kostengünstige Herstellung des Polstermoduls ermöglicht ist. Da ferner mittels des Polstermoduls mit dem offenzelligen Schaumstoff die gesamte Fläche des Polstermoduls bzw. des Füllmaterials als Messzone dient, werden somit Messlücken verringert und entsprechend die Messgenauigkeit erhöht. Ferner wird der Komfort erhöht, da das gesamte Füllmaterial aufgrund dessen Elastizität als Auflagefläche bzw. als Matratzenfläche dienen kann, ohne dass die Person aufgrund zusätzlicher Systemanordnungen harte und unkomfortable Bereiche spürt.
Als Fluid innerhalb des Innenvolumens kann beispielsweise ein gasförmiges Fluid, wie beispielsweise Luft oder ein Edelgas, oder ein flüssiges Fluid, wie beispielsweise Wasser, eingesetzt werden. Wird beispielsweise Wasser als Fluid eingesetzt, kann das Polstermodul zum Beispiel in Wasserbettsystemen eingesetzt werden.
Ferner kann eine Fluidpumpe mit dem Innenvolumen gekoppelt sein, um nach Bedarf Fluid in das Innenvolumen einzuströmen oder abzuzapfen, um einen gewünschten bestimmten Referenzdruck oder eine Referenzmasse des Fluides innerhalb des Innenvolumens herzustellen.
Die Ausleseeinheit weist beispielsweise einen Mikroprozessor auf, welcher beispielsweise kabelgebunden oder kabellos mit dem Sensor verbunden bzw. gekoppelt ist, um die Signale des Sensors zu erhalten. Die Ausleseeinheit wandelt die Signale beispielsweise in Übertragungsdaten eines gewünschten Formats um. Ferner kann die Ausleseeinheit ein Teil einer Steuereinheit sein.
Die Steuereinheit kann neben der Ausleseeinheit eine Sende- und Empfangseinheit aufweisen, welcher die gemessenen Massenstromsignale an eine externe Bedieneinrichtung sendet. die Bedieneinrichtung kann beispielsweise von einem Benutzer bedient werden. Die Bedieneinrichtung kann auch einen zentralen Computer darstellen, welcher z.B. Daten einer Vielzahl verschiedener (Patienten-)Betten, in welchen die oben beschriebenen Polstermodule eingesetzt werden, verarbeitet werden können. Die Bedieneinrichtung ist beispielsweise ein Tablet-Computer, ein Smartphone, ein Notebook oder ein Desktop-Computer, welche durch eine Person bedienbar sind. Die Steuereinheit kann kabelgebunden oder kabellos mit der Bedieneinrichtung verbunden sein.
Ferner kann die Steuereinheit mit der Fluidpumpe gekoppelt sein, so dass Steuersignale von der Steuereinheit an die Fluidpumpe übertragbar sind. Somit kann beispielsweise über die Bedieneinrichtung die Fluidpumpe gesteuert werden, um einen gewünschten Referenzdruck oder Referenzmasse des Fluides in dem Innenvolumen des Polstermoduls aktiv einzustellen. Ferner kann die Steuereinrichtung an eine Energieversorgungseinrichtung, beispielsweise mittels eines elektrischen Steckers, verbunden werden. Alternativ oder zusätzlich kann die Steuereinrichtung eine integrierte Stromversorgungseinrichtung, wie beispielsweise eine Batterie, aufweisen. Die Steuereinrichtung, die Ausleseeinheit und/oder der Sensor können innenliegend in dem Polstermodul angeordnet sein. Alternativ kann die Steuereinrichtung und/oder die Ausleseeinheit außerhalb des Polstermoduls angeordnet sein und über eine Datenverbindung, kabelgebunden oder kabellos, an den Sensor gekoppelt werden. Ferner kann der Sensor außerhalb des Polstermoduls angeordnet sein und, z.B. mittels einer Schlauchverbindung, mit dem Innenvolumen verbunden bzw. gekoppelt sein. Gemäß einer weiteren beispielhaften Ausführungsform ist der Sensor ausgebildet, eine Änderung der physikalischen Größe des Fluides einen Massenstrom) quantitativ zu messen. Der Sensor kann somit nicht nur feststellen, ob eine Änderung vorliegt, sondern kann auch die Stärke der Änderung, insbesondere über einen zeitlichen Verlauf, feststellen. Somit kann über die Zeit ein bestimmtes Bewegungsmuster der Person, beispielsweise während des Schlafens, erstellt werden, so dass basierend auf diesen Daten eine sichere Aussage getroffen werden kann, ob eine ungewöhnliche und möglicherweise gefährliche Position der Person oder eine normale Position der Person vorliegt. Somit kann die Anzahl an Fehlalarmen reduziert werden. Mittels einer quantitativen Messung des Sensors kann beispielsweise die Amplitudengröße und Frequenz der Änderungen ( Durchflussmengenänderungen) gemessen werden. Beispielsweise verursacht die Atmung eines Patienten, der in einem Bett liegt, eine spezifische Druckänderung und Druckverteilung in der Matratze allein aufgrund seiner Atmung. Wenn die Matratze aus zumindest einem der Polstermodule gebildet ist, kann die Atemfrequenz des Patienten gemessen werden. Ist die Atemfrequenz z.B. um eine vorbestimmte Anzahl an Hüben höher als normal kann auf eine Krankheit, wie z.B. Grippe oder epileptischer Anfall, geschlossen werden.
Gemäß einer weiteren beispielhaften Ausführungsform weist die Polstervorrichtung ferner ein weiteres Koppelelement, welches mit dem Innenvolumen gekoppelt ist, auf, wobei an das weitere Koppelelement der Sensor und/oder ein weiterer Sensor derart ankoppelbar ist, dass mittels des Sensors und/oder des weiteren Sensors die Änderung (z.B. Druckänderung oder Massenstromänderung) der physikalischen Größe (z.B. Druck oder Massenstrom) des Fluides in dem Innenvolumen messbar ist. Das weitere Koppelelement ist beabstandet von dem Koppelelement angeordnet.
Mittels des Einsatzes weiterer Koppelelemente bzw. Sensoren in einem Polstermodul können beispielsweise eine Vielzahl von lokalen Änderungen (z.B. Druckänderungen) über die gesamte Liegefläche des Polstermoduls und somit ein Änderungsverteilungsmuster (z.B. ein Druckverteilungsmuster) über die Fläche des Polstermoduls gemessen werden. Somit kann eine bestimmte Liegeposition des Patienten festgestellt werden. So kann dadurch beispielsweise festgestellt werden, ob der Patient flach mit seinem Rücken auf dem Polstermodul auffliegt, seitlich bzw. in einer Seitenposition auf dem Polstermodul auffliegt, oder ob der Patient auf dem Polstermodul sitzt. Dies ist möglich, da jede Liegeposition eine charakteristische Änderung (Druckänderung) der physikalischen Größe des Fluides (Druckverteilung) entlang des Sitzpolsters aufweist. Die charakteristische Änderung/ Druckverteilung für jede Liegeposition kann beispielsweise in Laborversuchen festgestellt werden und anschließend als Soll-Änderungs-(Druck)verteilung für die entsprechende Liegeposition dem System vorgegeben werden.
Gemäß einer weiteren beispielhaften Ausführungsform weist das Polstermodul ferner eine erste Kammer mit dem Füllmaterial und eine zweite Kammer mit einem weiteren Füllmaterial auf. Die erste Kammer und die zweite Kammer sind fluiddicht zueinander ausgebildet. Das weitere Füllmaterial kann beispielsweise aus dem oben beschriebenen offenzelligen Schaumstoff oder aus einem anderen elastisch verformbaren Material, wie beispielsweise aus Federn oder Kunststoff, bestehen.
Gemäß einer weiteren beispielhaften Ausführungsform ist das Koppelelement an die ersten Kammer gekoppelt und das weiterer Koppelelement an die zweite Kammer gekoppelt. Beispielsweise ist der Sensor mittels des Koppelelements an die erste Kammer gekoppelt, wobei ein weiterer Sensor mittels des weiteren Koppelelements an die zweite Kammer gekoppelt ist, wobei der weitere Sensor ebenfalls mit der Ausleseeinheit gekoppelt ist. Mittels obiger Anordnung kann über das gesamte Polstermodul ein noch genaueres Änderungs- (Druck-)verteilungsmuster gemessen werden.

Ferner kann der Sensor nicht-erfindungsgemäß derart angeordnet sein, dass der Sensor in Fluidverbindung mit der ersten Kammer und der zweiten Kammer steht. So kann beispielsweise der Sensor in einem Koppelbereich zwischen der ersten Kammer und der zweiten Kammer angeordnet sein und somit mit einer ersten Druckseite den Druck in der ersten Kammer messen und mit einer zweiten Druckseite den Druck in der zweiten Kammer messen. Ferner kann somit der Sensor den gemessenen Druck in der ersten Kammer mit dem gemessenen Druck in der zweiten Kammer vergleichen und somit eine Druckbelastung der jeweiligen Kammer des Polstermoduls bestimmen.

Ferner kann der Sensor als Durchflusssensor ausgebildet werden. Die erste Kammer und die zweite Kammer des Polstermoduls können einen Übergangsbereich aufweisen, entlang welchem das Fluid zwischen der ersten Kammer und der zweiten Kammer ausgetauscht wird. Der Sensor als Durchflusssensor kann den Austausch bzw. den Massenstrom des Fluids zwischen der ersten Kammer und der zweiten Kammer messen. Basierend auf dem gemessenen Fluss des Massenstroms des Fluids kann ebenfalls auf eine Druckbelastung des Polstermoduls geschlossen werden. Der Übergangsbereich kann beispielsweise mittels eines Ventils oder eines Verbindungsschlauchs gebildet werden.

Der Sensor als Durchflusssensor kann ebenfalls an einer Öffnung des Polstermoduls angeordnet sein, wobei die Öffnung eine Fluidverbindung zwischen dem Innenvolumen und der Umgebung des Polstermoduls bereitstellt. In der Öffnung kann ferner ein Ventil angeordnet sein, sodass bei Druckbelastung des Polstermoduls das Fluid, wie beispielsweise Luft, aus dem Innenvolumen in die Umgebung ausströmen. Dieses Ausströmen des Fluids wird durch den Sensor als Durchflusssensor gemessen, sodass entsprechend die Belastung des Polstermoduls gemessen wird.

Das Polstermodul kann ferner elastisch verformbar ausgebildet werden, indem das Polstermodul selbst aus einem elastisch verformbaren Material hergestellt wird oder indem der offenzellige Schaumstoff im Innenvolumen des Polstermoduls elastisch verformbare Eigenschaften aufweist. Somit kann bei Entlastung des Polstermoduls dieses wieder in seine ursprüngliche Form zurückverformt werden. In diesem Fall kann durch die oben beschriebene Öffnung das Fluid, wie beispielsweise Luft, erneut in das Innenvolumen von der Umgebung oder von einer benachbarten Kammer einströmen. Dieses Einströmen des Fluids in das Innenvolumen kann entsprechend mittels des Sensors als Durchflusssensor gemessen werden, sodass die hieraus ergebende Belastungsänderung mittels des Sensors messbar ist.

Gemäß einem nicht-erfindungsgemäßen Aspekt der vorliegenden Erfindung wird ein System zum Überwachen von Personen-/Patientenbewegungen beschrieben. Das System weist ein erstes Polstermodul, welches z.B. entsprechend dem oben beschriebenen Polstermodul ausgebildet ist, auf. Auf das erste Polstermodul ist zumindest ein erster Anteil eines Körpergewichts eines Patienten/einer Person übertragbar. Das erste Polstermodul weist ein erstes fluiddichtes Innenvolumen auf, sodass bei Änderung einer Belastung des ersten Polstermoduls eine Änderung (Druckänderung) einer physikalischen Größe eines Fluides innerhalb des Polstermoduls generierbar ist. Ferner weist das System ein zweites Polstermodul, welches z.B. entsprechend dem oben beschriebenen Polstermodul ausgebildet ist, auf. Auf das zweite Polstermodul ist zumindest ein zweiter Anteil eines Körpergewichts eines Patienten übertragbar.
Das erste Polstermodul weist ein erstes Anschlusselement und das zweite Polstermodul weist ein zweites Anschlusselement auf, wobei das erste Anschlusselement und das zweite Anschlusselement derart ausgebildet sind, dass das erste Anschlusselement lösbar an das zweite Anschlusselement ankoppelbar ist, um das erste Polstermodul lösbar an dem zweiten Polstermodul zu befestigen. Zumindest das erste Polstermodul weist ein Koppelelement auf, welches mit dem ersten Innenvolumen gekoppelt ist, wobei an das Koppelelement ein erster Sensor derart ankoppelbar ist, dass mittels des ersten Sensors eine Änderung (Druckänderung) der physikalischen Größe des Fluides in dem Innenvolumen messbar ist.
Das erste Polstermodul und das zweite Polstermodul können beispielsweise wie das beschriebene Polstermodul der Polstervorrichtung ausgebildet werden.

So kann das erste Polstermodul und/oder das zweite Polstermodul beispielsweise einen elastischen offenzelligen Schaumstoff mit fluiddurchlässigen Zellwänden aufweisen. Ferner können das erste Polstermodul und/oder das zweite Polstermodul fluiddichte Kammern aufweisen.

Das erste Anschlusselement und das zweite Anschlusselement können beispielsweise zusammen eine Reißverschlussverbindung, eine Klettverbindung oder eine Knopfverbindung ausbilden, um das erste Polstermodul lösbar an dem zweiten Polstermodul zu befestigen. Ferner kann das erste Anschlusselement und das zweite Anschlusselement eine Steckverbindung oder eine Schraubverbindung ausbilden. Das erste Anschlusselement kann beispielsweise einen Gewindeflansch aufweisen und das zweite Anschlusselement kann einen Flansch mit einer drehbaren Mutter aufweisen, welche auf den Gewindeflansch aufschraubbar ist. Ferner kann das erste Anschlusselement und das zweite Anschlusselement eine Bajonettverbindung ausbilden. Zudem kann das erste Anschlusselement einen ersten Magneten und das zweite Anschlusselement einen zweiten Magneten aufweisen, wobei der erste Magnet und der zweite Magnet derart relativ zueinander gepolt sind, dass sich beide Magneten gegenseitig anziehen, um somit eine Haltekraft zwischen den Polstermodulen zu erzeugen.

Aufgrund der Ausbildung der entsprechenden Anschlusselemente an den entsprechenden Polstermodulen, kann ein modulares System zur Überwachung von Bewegungen einer Person bereitgestellt werden. Beispielsweise kann eine Vielzahl von einheitlichen und normierten Polstermodulen hergestellt werden, welche alle z.B. die gleiche Dimensionierung aufweisen. Je nach Anwendungsfall, beispielsweise als Liegematratze oder als Sitzkissen, können entsprechend viele Polstermodule mittels entsprechenden Anschlusselementen modular und lösbar befestigt werden. Der Sensor in zumindest einem Polstermodul kann somit eine Änderung (Druckänderung) der physikalischen Größe des Fluides des Polstermoduls, beispielsweise aufgrund einer Bewegung einer Person, messen. Somit ist aufgrund der modularen Ausbildung ein einfach herzustellendes und gleichzeitig flexibel einsetzbares Überwachungssystem für Personen bereitgestellt.

Gemäß einer weiteren beispielhaften Ausführungsform weist das zweite Polstermodul ein zweites fluiddichtes Innenvolumen auf, sodass bei Änderung einer Belastung des zweiten Polstermoduls eine Änderung (Druckänderung) der physikalischen Größe des Fluides innerhalb des zweiten Polstermoduls generierbar ist.

Gemäß einer weiteren beispielhaften Ausführungsform verbindet das zweite Anschlusselement das zweite Innenvolumen selektiv mit einer zweiten Umgebung des zweiten Polstermoduls, wobei das erste Anschlusselement und das zweite Anschlusselement derart gekoppelt sind, dass ein Fluidaustausch zwischen dem ersten Innenvolumen und dem zweiten Innenvolumen bereitstellbar ist. Mittels der beispielhaften Ausführungsform kann das erste Anschlusselement und das zweite Anschlusselement eine fluiddichte Verbindung zwischen dem ersten Innenvolumen und dem zweiten Innenvolumen herstellen. Somit beeinflusst eine Änderung (Druckänderung) der physikalischen Größe des Fluides in dem ersten Polstermodul eine Änderung (Druckänderung) der physikalischen Größe des Fluides in dem zweiten Polstermodul. Somit können mehrere Polstermodule miteinander befestigt werden, wobei es ausreicht zumindest einen Sensor in einem der Polstermodule anzuordnen. Aufgrund der Fluidverbindung zwischen den Polstermodulen ist eine Druckänderung mit dem Sensor messbar, unabhängig an welcher Stelle und welches Polstermodul mit Druck belastet wird.

Das erste Anschlusselement und das zweite Anschlusselement können beispielsweise jeweils ein Ventil aufweisen, welches erst in einem fluiddurchlässigen Zustand einstellbar ist, wenn die Anschlusselemente miteinander verbunden sind, so dass kein Fluid aus dem entsprechenden Innenvolumen eines Polstermoduls in die Umgebung der Polstermodule ausströmen kann.

Gemäß einer weiteren beispielhaften Ausführungsform weist das zweite Polstermodul ein weiteres Koppelelement auf, welches mit dem zweiten Innenvolumen gekoppelt ist. An das weitere Koppelelement ist ein zweiter Sensor derart ankoppelbar, dass mittels des zweiten Sensors eine Druckänderung in dem zweiten Innenvolumen messbar ist. Wenn verschiedene Polstermodule oder alle Polstermodule jeweils zumindest ein Koppelelement bzw. einen Sensor aufweisen, kann ein Druckverteilungsmuster über die gesamte Fläche der entsprechenden Polstermodule gemessen werden und somit, wie oben beschrieben, z.B. eine bestimmte Liegeposition oder eine gewünschte andere vordefinierte Position der Person bestimmt werden. Der Sensor kann beispielsweise zur Übertragung der Signale an eine weitere zweite Ausleseeinheit des zweiten Polstermoduls gekoppelt werden. Alternativ können alle Sensoren an eine gemeinsame Ausleseeinheit gekoppelt werden.

Gemäß einer weiteren beispielhaften Ausführungsform weist das System ein drittes Polstermodul auf, auf welches zumindest ein dritter Anteil des Körpergewichts der Person übertragbar ist. Das erste Polstermodul weist ein weiteres erstes Anschlusselement und das dritte Polstermodul weist ein drittes Anschlusselement auf. Das weitere erste Anschlusselement und das dritte Anschlusselement sind derart ausgebildet, dass das weitere erste Anschlusselement lösbar an das dritte Anschlusselement ankoppelbar ist, um das erste Polstermodul lösbar mit dem dritten Polstermodul zu befestigen.

Mit dem obigen Ausführungsbeispiel wird verdeutlicht, dass ein Polstermodul eine Vielzahl von Anschlusselementen aufweisen kann. Beispielsweise kann das erste Polstermodul an gegenüberliegenden Seitenflächen oder an allen Seitenflächen entsprechende Anschlusselemente aufweisen. Somit kann eine beliebige Vielzahl von weiteren Polstermodulen an das erste Polstermodul gekoppelt werden. Ferner können die Polstermodule an beliebigen Stellen miteinander verbunden werden, so dass beliebige Muster von Polstermodulen gebildet werden können.

Gemäß einer weiteren beispielhaften Ausführungsform weist zumindest das erste Polstermodul in dem ersten fluiddichten Innenvolumen ein oben beschriebenes Füllmaterial aus einem offenzelligen Schaumstoff auf, wobei der offenzellige Schaumstoff fluiddurchlässige Zellwände aufweist. Das Füllmaterial ist mit einer Umhüllung fluiddicht umhüllt, sodass bei Änderung einer Belastung des ersten Polstermoduls eine Druckänderung innerhalb des ersten Polstermoduls generierbar ist.

Mit der vorliegenden Erfindung wird ein einfach herzustellendes und sehr anpassbares System zum Überwachen von Bewegungen einer Person beschrieben. Es werden Polstermodule bereitgestellt, welche einen offenzelligen Schaumstoff als Füllmaterial aufweisen, welcher luftdicht beschichtet bzw. verpackt ist, sodass bei Belastung ein Innendruck entsteht.

Das System ist ferner modular ausgebildet, wobei zumindest zwei Polstermodule lösbar gekoppelt werden können. Verschiedene Polstermodule (z.B. Elemente, welche mittels Belastung von Hohlräumen (Innenvolumen) ein Drucksignal generieren z.B. in Sesseln, Sesselauflagen, Matten, Matratzen etc.) können an eine elektronische Hardware (z.B. Ausleseeinheit der Steuereinheit) angeschlossen werden. Diese Ausleseeinheit kann mittels unterschiedlich abgestufter Software bzw. Applikationen (sog. Apps) für verschiedene Anwendungsbereiche verwendet werden und das Drucksignal situationsbezogen interpretieren.

Im Folgenden werden beispielhaft Einsatzmöglichkeiten einiger beispielhafter Ausführungsformen des erfindungsgemäßen Systems beschrieben.
Die Verwendung eines Sensors, welcher quantitativ den Druck misst, und mittels der Weiterverarbeitung der Messdaten in der Steuereinheit wird eine flexible Interpretation des Drucksignals über die Zeit ermöglicht. Die entstehenden Änderungen können mittels der Sensoren kontinuierlich gemessen und über unterschiedliche Algorithmen interpretiert werden. Die Daten können kabelgebunden oder kabellos zur Verarbeitung weitergegeben werden. Verschiedene Hard- bzw. Software- Abstufungen sind möglich (W-LAN, Cloud und Einspeisung in die elektronische Patientenakte, bzw. lokal an eine Rufanlage).
Beispielsweise kann ein Alarm ausgelöst werden, wenn der Druck durch das Verlassen des Polstermoduls sinkt. Ferner wird das Polstermodul z.B. automatisch "aktiviert", wenn das Polster z.B. mindestens 5 min durchgehend belastet wurde.
Ferner können die Polstermodule des Systems als Sturzmatten eingesetzt werden und neben einem Bett auf dem Boden angeordnet werden (Druck "hoch" bei Betreten oder Herausfallen einer solchen Sturzmatte).
Die Polstermodule können als Randbereiche einer Matratze eingesetzt werden, so dass ein Alarm ausgelöst wird, wenn plötzlich der Druck steigt und anschließend wieder stark reduziert wird. Dies könnte ein Herausfallen des Patienten implizieren.
Die Polstermodule können als Mittelbereiche einer Matratze eingesetzt werden, so dass ein Alarm ausgelöst wird, wenn plötzlich der Druck sinkt. Dies könnte ebenfalls ein Herausfallen des Patienten implizieren. Ein Alarm kann ausgelöst werden, wenn der Druck durch das Verlassen des Polstermoduls sinkt. Die Matratze wird automatisch "aktiviert", wenn diese z.B. mindestens 5 min durchgehend belastet wurde.

Die Polstermodule können z.B. an einen Lattenrost befestigt werden und sind dann Teil eines Betts.

Die Polstermodule können z.B. auch an einem Matratzenüberzug befestigt werden und sind dann Teil der Matratze.

Durch das Messen eines Druckverteilungsmusters innerhalb eines Polstermoduls und/oder über mehrerer zusammengeschlossener Polstermodule können z.B. folgende Basisinformation erhalten werden.

Ist das Polstermodul ein Sitzkissen/-polster, erhält man folgende Informationen:
i.) Person sitzt (Druck hoch)
ii.) Person steht auf (Druck tief)

Ist das Polstermodul eine Sturzmatte, erhält man folgende Informationen:
i.) Matte belastet, Person steht / bzw. ist aus dem Bett gefallen (Druck hoch)
ii.) Matte nicht belastet (Druck tief)

Bildet das Polstermodul oder die Polstermodule eine Matratze, erhält man folgende Informationen:
i.) Person liegt im Bett und schläft (Mittleres Segment, Druck hoch)
ii.) Person liegt im Bett und ist wach (Mittleres Segment, Druck hoch jedoch wechselnd)
iii.) Person steht auf (Mittleres Segment, Druck tief) oder
iv.) Person steht auf (Randsegment, Druck hoch) optional Bildet das Polstermodul oder die Polstermodule ein Bett, erhält man folgende Informationen:
   i) Person liegt im Bett und schläft (Druck hoch)
   ii) Person liegt im Bett und ist wach (Druck hoch jedoch wechselnd)
   iii) Person steht auf (Druck tief)

Ferner besteht mit dem System die Möglichkeit gefährliche Situationen (z.B. einen Sturz einer Person) zu erfassen. Dabei sind Informationen bzgl. der Veränderung des Aufenthaltsorts messbar. Informationen, wie dass Verlassen des Bettes/Matratze oder eines Sessels, stellen hierfür zentrale Informationen dar.
Ferner werden Patienten, welche starke auf Morphinbasis bestehende Analgetika konsumieren müssen, oft mittels eines Pulsoxymeters überwacht.
Dieses misst mittels Lichtabsorption (meist Finger) die arterielle Sauerstoffsättigung, um einen eventuellen Atemstillstand detektieren zu können. Durch Fingerbewegungen werden jedoch Fehlalarme ausgelöst und stören so den Routineablauf im Krankenhaus. Atembewegungen übertragen sich durch die Matratze (Polstermodul) fort und werden als minimale Druckschwankungen registriert. Diese fast unscheinbaren Bewegungen können mittels der vorliegenden Erfindung analysiert werden (Druckverteilungsmuster, Druckänderungskurve, Frequenz). So kann bei fehlenden Druckschwankungen auf einen Atemstillstand geschlossen- und ein Alarm generiert werden noch bevor das Pulsoxymeter diesen durch eine resultierende und damit später auftretende geringere Sauerstoffsättigung auslösen kann.

Ferner können z.B. epileptische Anfälle detektiert werden. Dieser kann durch einen dem Rhythmus und der Intensität entsprechenden Druckverlauf erkannt werden (Druckänderungskurve hat z.B. eine spezifische Charakteristik).

Ferner kann das erfindungsgemäße System in der Hauskrankenpflege eingesetzt werden. Ist eine betreute Person z.B. 20% länger als üblich im Bett (Selbstadaptives System; z.B. wenn Druck eines mittleren Segments eines Polstermoduls nach vorbestimmter Zeitdauer immer noch hoch ist), sodass die Betreuungsperson automatisch informiert werden kann.

Wie bereits oben erläutert, verursacht die Atmung einer Person, der in einem Bett liegt, eine spezifische Druckänderung und Druckverteilung in der Matratze. Wenn die Matratze aus zumindest einem der Polstermodule gebildet ist, kann die Atemfrequenz der Person gemessen werden. Ist die Atemfrequenz z.B. um eine vorbestimmte Anzahl an Hüben höher als normal kann auf eine Krankheit, wie z.B. Grippe, geschlossen werden,

Bei keinen bzw. wenig Positionsveränderungen der Person über die Zeit wird eine Information generiert. Bewegungen, wie Gesäßentlastung oder Drehen, werden in der Druckänderungskurve sichtbar. Wird eine bestimmte Maximalzeit überschritten kann die Pflegeperson automatisch informiert werden. So kann z.B. Dekubitus vorgebeugt werden.

Ferner kann die Atemfrequenz und z.B. der Puls, welcher ebenfalls eine Druckänderung in dem Polstermodul verursacht, aufgezeichnet werden, um Daten von Personen (z.B. Sportlern), im Ruhzustand aufzuzeichnen. Im Ruhezustand wird somit ein Ruhepuls, eine Atemfrequenz, eine Schlafintensität, eine Durchschnittsschlafdauer etc. aufgezeichnet. Die Daten können von der Steuereinheit als Weblösung im Internet aufgezeichnet und abgerufen werden. Einmal eingeschaltet liefert die Steuereinheit z.B. über WLAN-Daten via Internet an ein Aufzeichnungsgerät.

Ferner kann das System in einem Schlaflabor eingesetzt werden, um die Schlafqualität zu messen. Informationen über die Liegedauer und die Schlafqualität der Person können das klinische Gesamtbild erweitern. Es wird darauf hingewiesen, dass die hier beschriebenen Ausführungsformen lediglich eine beschränkte Auswahl an möglichen Ausführungsvarianten der Erfindung darstellen. So ist es möglich, die Merkmale einzelner Ausführungsformen in geeigneter Weise miteinander zu kombinieren, so dass für den Fachmann mit den hier expliziten Ausführungsvarianten eine Vielzahl von verschiedenen Ausführungsformen als offensichtlich offenbart anzusehen sind. Insbesondere sind einige Ausführungsformen der Erfindung mit Vorrichtungsansprüchen und andere Ausführungsformen der Erfindung mit Verfahrensansprüchen beschrieben. Dem Fachmann wird jedoch bei der Lektüre dieser Anmeldung sofort klar werden, dass, sofern nicht explizit anders angegeben, zusätzlich zu einer Kombination von Merkmalen, die zu einem Typ von Erfindungsgegenstand gehören, auch eine beliebige Kombination von Merkmalen möglich ist, die zu unterschiedlichen Typen von Erfindungsgegenständen gehören, im Rahmen der vorliegenden Ansprüche.

### Kurze Beschreibung der Zeichnungen

Im Folgenden werden zur weiteren Erläuterung und zum besseren Verständnis der vorliegenden Erfindung Ausführungsbeispiele unter Bezugnahme auf die beigefügten Zeichnungen näher beschrieben. Es zeigen:
Fig. 1 eine schematische Darstellung eines Systems mit Polstermodulen gemäß einer beispielhaften Ausführungsform der vorliegenden Erfindung;
Fig. 2 eine schematische Darstellung eines Systems mit weiteren Polstermodulen gemäß einer beispielhaften Ausführungsform der vorliegenden Erfindung;
Fig. 3 eine schematische Darstellung einer Matratze, welche aus mehreren Polstermodulen gemäß einer beispielhaften Ausführungsform der vorliegenden Erfindung hergestellt ist; und
Fig. 4 eine schematische Darstellung eines Verfahrens gemäß einer beispielhaften Ausführungsform der vorliegenden Erfindung.

### Detaillierte Beschreibung von exemplarischen Ausführungsformen

Gleiche oder ähnliche Komponenten in unterschiedlichen Figuren sind mit gleichen Bezugsziffern versehen. Die Darstellungen in den Figuren sind schematisch.

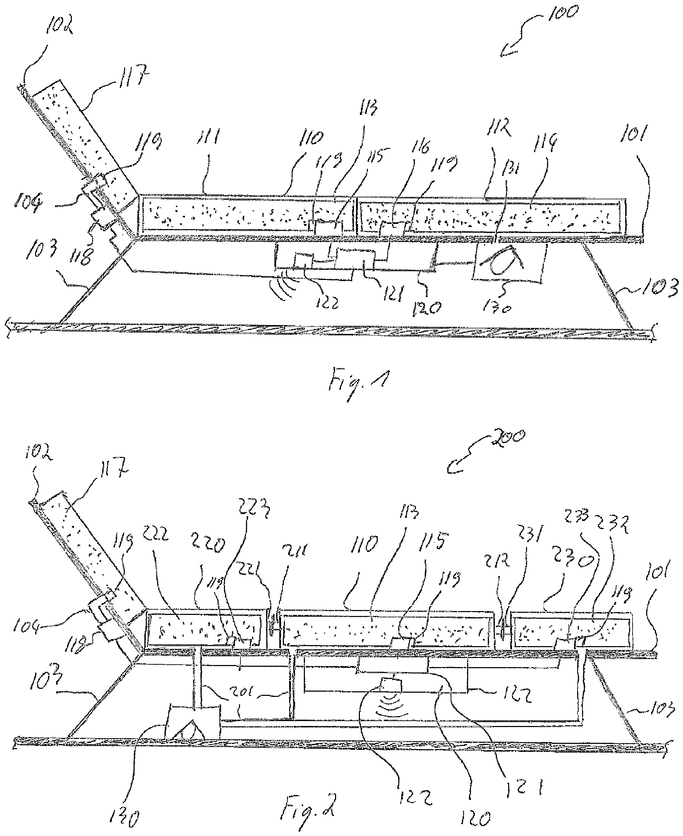

**Fig. 1** zeigt eine Polstervorrichtung 100 zum Überwachen von Bewegungen einer Person. Auf einem Polstermodul 110 ist zumindest ein Anteil eines Körpergewichts einer Person übertragbar, wobei das Polstermodul 110 ein Innenvolumen aufweist, in welchem ein Füllmaterial 113 aus einem offenzelligen Schaumstoff angeordnet ist. Der offenzellige Schaumstoff weist fluiddurchlässige Zellwände auf, wobei das Füllmaterial 113 mit einer Umhüllung fluiddicht umhüllt ist, sodass bei Änderung einer Belastung des Polstermoduls 110 eine Änderung einer physikalischen Größe eines Fluides innerhalb des Polstermoduls 110 generierbar ist. Ein Koppelelement 119 ist mit dem Innenvolumen gekoppelt, wobei an das Koppelelement 119 ein Sensor 115 derart ankoppelbar ist, dass mittels des Sensors 115 eine Änderung der physikalischen Größe des Fluides in dem Innenvolumen messbar ist. Eine Ausleseeinheit 121 ist z.B. mit dem Sensor 115 derart gekoppelt, dass Signale, welche indikativ für die Änderung der physikalischen Größe des Fluides sind, von der Ausleseeinheit 121 auslesbar sind.
Das Koppelelement 119 kann beispielsweise ein Ventil, einen Verschluss oder eine Öffnung aufweisen, woran ein entsprechender Sensor 115, 116 angekoppelt werden kann. Ferner kann das Koppelement 119 derart ausgebildet sein, dass ein Sensor 118 außerhalb des Polstermoduls 110 angeordnet werden kann und mittels eines Verbindungsschlauchs 104, welcher an dem Koppelement 119 (lösbar) gekoppelt ist, angekoppelt werden kann. Die Sensoren 115, 116 in Fig. 1 sind im Inneren des Polstermoduls 110 angeordnet. Das weitere Polstermodul 117 in Fig. 1 zeigt eine alternative Ausführungsform, in welcher ein Sensor 118 außerhalb des weiteren Polstermoduls 117 angeordnet ist.
Das Polstermodul 110, 117 bildet wie in Fig.1 dargestellt beispielsweise eine Matratze eines Betts 101 aus. Das Polstermodul 110, 117 ist elastisch verformbar ausgebildet und stellt eine weiche Auflagefläche für einen Körper, bzw. einen Patienten/Person, dar. Der Patient kann sich beispielsweise auf das Polstermodul 110, 117 legen, stellen oder setzen und zumindest einen Anteil seines Körpergewichts oder sein gesamtes Körpergewicht auf das Polstermodul 110, 117 übertragen.
Das Polstermodul 110 weist insbesondere ferner den Sensor 115, 116 zur Messung der Änderung der physikalischen Größe des Fluides auf, welcher in dem Polstermodul 110 (bzw. in den entsprechenden Kammern 111, 112 des Polstermoduls 110) angeordnet ist und mittels des entsprechenden Koppelelements 119 an das entsprechende Innenvolumen gekoppelt ist. Der Sensor 115, 116 ist mit der Ausleseeinheit 121 derart koppelbar, dass Signale, welche indikativ für die Änderung der physikalischen Größe des Fluides sind, von der Ausleseeinheit 121 auslesbar sind.
Der Sensor 115, 116 kann direkt im Innenvolumen des Polstermoduls 110 angeordnet sein oder in einem Zwischenbereich zwischen dem Innenvolumen und einer Außenhülle des Polstermoduls 110. Das Koppelelement 119 kann z.B. eine Fixierung des Sensors 115, 116 im Innenvolumen oder im Zwischenbereich ermöglichen, z.B. mittels einer Klettverbindung.
Ein Sensor 118 der Polstervorrichtung 100 kann zudem oder alternativ außerhalb des weiteren Polstermoduls 117 angeordnet werden und mittels des Koppelelements 119 an das Innenvolumen gekoppelt werden. Der Sensor 118 ist mit einer Ausleseeinheit 121 derart koppelbar, dass Signale, welche indikativ für die Änderung der physikalischen Größe des Fluides sind, von der Ausleseeinheit 121 auslesbar sind.
Wird der Sensor 118 außerhalb des Polstermoduls 117 angeordnet, kann das Polstermodul 117 ohne elektrische Bauteile/Sensoren hergestellt und vertrieben werden. Das Polstermodul 117 ist dann über den Verbindungsschlauch 104 mit dem Sensor 118 bzw. der Steuereinheit 120 verbunden.
Der Sensor 118 kann beispielsweise in einem Steuergehäuse bzw. einer Steuereinheit 120 angeordnet werden, welche außerhalb des Polstermoduls 117 angeordnet ist. Die Steuereinheit 120 ist beispielsweise an einem Bett 101 befestigt, auf welchem ein Polstermodul 110, 117 aufliegt. Der Sensor 118 ist mit dem Verbindungsschlauch 104 mit dem Koppelelement 119 (z.B. Ventil) des Polstermoduls 110, 117 (lösbar) angekoppelt, sodass eine Änderung (Druckänderung) der physikalischen Größe des Fluides im Innenvolumen des Polstermoduls 117 von dem Sensor 110 messbar ist.

Im Inneren des Polstermoduls 110 ist das Innenvolumen ausgebildet. In dem Innenvolumen kann beispielsweise ein weiches Füllmaterial 113 vorliegen. Das Füllmaterial 113 kann ganz oder bereichsweise aus einem offenzelligen (bzw. offenporigen) Schaumstoff bestehen, wobei der offenzellige Schaumstoff fluiddurchlässige Zellwände aufweist. Der offenzellige Schaumstoff ist zusammendrückbar, um mittels der Druckbelastung sein Volumen zu verkleinern. Bei offenzelligen Schaumstoffen sind die Zellwände nicht geschlossen, so dass diese ein Fluid aufnehmen und durchlassen können.

Das Füllmaterial 113 in dem Polstermodul 110 ist mit einer Umhüllung fluiddicht umhüllt. Die Umhüllung kann beispielsweise als fluiddichte Folie ausgebildet sein. Wird das Polstermodul 110 und entsprechend das Innenvolumen bzw. das Füllmaterial 113 durch Belastung der Person zusammengedrückt, so ändert sich der Druck im Inneren des Innenvolumens bzw. des Füllmaterials 113.

Als Fluid innerhalb des Innenvolumens kann beispielsweise ein gasförmiges Fluid, wie beispielsweise Luft oder ein Edelgas, oder ein flüssiges Fluid, wie beispielsweise Wasser, eingesetzt werden.

Ferner kann eine Fluidpumpe 130 mit dem Innenvolumen gekoppelt sein, um nach Bedarf Fluid in das Innenvolumen einzuströmen oder abzuzapfen, um einen gewünschten bestimmten Referenzdruck innerhalb des Innenvolumens herzustellen. Die Fluidpumpe 130 kann in dem Polstermodul 110 integriert sein oder außerhalb an dem Bettgestell 103 des Betts 101, wie in Fig. 1 dargestellt, angeordnet werden. Zwischen der Fluidpumpe 130 und dem Polstermodul 110 können Versorgungsschläuche angeordnet werden.

Die Ausleseeinheit 121 weist beispielsweise einen Mikroprozessor auf, welcher beispielsweise kabelgebunden oder kabellos mit dem Sensor 115 verbunden bzw. gekoppelt ist, um die Signale des Sensors 115 zu erhalten. Die Ausleseeinheit 121 wandelt die Signale beispielsweise in Übertragungsdaten eines gewünschten Formats um. Ferner kann die Ausleseeinheit 121 ein Teil einer Steuereinheit 120 sein.
Die Steuereinheit 120 kann neben der Ausleseeinheit 121 eine Sende- und Empfangseinheit 122 aufweisen, welche die gemessenen Drucksignale an eine externe Bedieneinrichtung sendet. Ferner kann die Steuereinheit 120 mit der Fluidpumpe 130 gekoppelt sein, so dass Steuersignale von der Steuereinheit 120 an die Fluidpumpe 130 übertragbar sind. Somit kann beispielsweise über die Bedieneinrichtung die Fluidpumpe 130 gesteuert werden, um einen gewünschten Referenzdruck in dem Innenvolumen des Polstermoduls 110 aktiv einzustellen.
Die Steuereinrichtung 120 oder zumindest die Ausleseeinheit 121 kann außerhalb des Polstermoduls 110, z.B. am Bettgestell 103 des Betts 101, angeordnet sein und über eine Datenverbindung, kabelgebunden oder kabellos, an den Sensor 115, 116, 118 gekoppelt werden.
Das Polstermodul 110 kann, wie in Fig. 1 dargestellt, zumindest einen weiteren Sensor 116 aufweisen, welcher beabstandet zu dem Sensor 115 angeordnet ist. Der weitere Sensor 116 ist mit der Ausleseeinheit 121 gekoppelt. Ferner kann ein weiteres Polstermodul 117 mit dem weiteren Sensor 118 an einer Kopfstütze 102 des Betts 101 angeordnet sein.
Mittels des Einsatzes weiterer Sensoren 116, 118 können beispielsweise eine Vielzahl von lokalen Änderungen der physikalischen Größe des Fluides über die gesamte Liegefläche des Polstermoduls 110 und somit ein (Druck-)Verteilungsmuster über die Fläche des Polstermoduls 110 gemessen werden. Somit kann eine bestimmte Liegeposition der Person festgestellt werden.

Das Polstermodul 110 weist in Fig. 1 beispielhaft ferner eine erste Kammer 111 mit dem Füllmaterial 113 und eine zweite Kammer 112 mit einem weiteren Füllmaterial 114 auf. Die erste Kammer 111 und die zweite Kammer 112 sind fluiddicht zueinander ausgebildet. Das weitere Füllmaterial 114 kann beispielsweise aus dem oben beschriebenen offenzelligen Schaumstoff oder aus einem anderen elastisch verformbaren Material, wie beispielsweise aus Federn oder Kunststoff, bestehen.
Der Sensor 115 ist in der ersten Kammer angeordnet, wobei der weitere Sensor 116 in der zweiten Kammer 112 angeordnet ist. Der weitere Sensor 116 ist mit der Ausleseeinheit 121 gekoppelt. Mittels dieser Anordnung kann über das gesamte Polstermodul 110 ein genaues Druckverteilungsmuster gemessen werden.
**Fig. 2** zeigt einer weitere beispielhafte Ausführungsform des erfindungsgemäßen Systems 100. Das System 200 weist ein erstes Polstermodul 110, welches z.B. entsprechend dem in Fig. 1 beschriebenen Polstermodul 110 ausgebildet ist, auf. Auf das erste Polstermodul 110 ist zumindest ein erster Anteil eines Körpergewichts einer Person übertragbar. Das erste Polstermodul 110 weist ein erstes fluiddichtes Innenvolumen auf, sodass bei Änderung einer Belastung des ersten Polstermoduls 110 eine Änderung der physikalischen Größe des Fluides innerhalb des ersten Polstermoduls 110 generierbar ist.
Ferner weist das System 200 ein zweites Polstermodul 220, welches z.B. entsprechend dem oben beschriebenen Polstermodul 110 ausgebildet ist, auf. Auf das zweite Polstermodul 220 ist zumindest ein zweiter Anteil eines Körpergewichts einer Person übertragbar.
Das erste Polstermodul 110 weist ein erstes Anschlusselement 211 und das zweite Polstermodul 220 weist ein zweites Anschlusselement 221 auf, wobei das erste Anschlusselement 211 und das zweite Anschlusselement 221 derart ausgebildet sind, dass das erste Anschlusselement 211 lösbar an das zweite Anschlusselement 221 ankoppelbar ist, um das erste Polstermodul 110 lösbar an dem zweiten Polstermodul 220 zu befestigen.
Zumindest das erste Polstermodul 110 weist ein Koppelelement 119 auf, welches mit dem ersten Innenvolumen gekoppelt ist, wobei an das Koppelelement 119 ein erster Sensor 115 derart ankoppelbar ist, dass mittels des ersten Sensors 115 eine Änderung der physikalischen Größe des Fluides in dem Innenvolumen messbar ist.
Beispielsweise weist das erste Polstermodul 110 den ersten Sensor 115 zur Messung der Änderung der physikalischen Größe des Fluides auf. Ferner weist das System 200 die Ausleseeinheit 121 auf, welche mit dem ersten Sensor 115 derart gekoppelt ist, dass Signale, welche indikativ für die Änderung der physikalischen Größe des Fluides sind, von der Ausleseeinheit 121 auslesbar sind.
Der erste Sensor 115 kann beispielsweise in einem Übergangsbereich zwischen dem ersten Polstermodul 110 und dem zweiten Polstermodul 220 angeordnet sein. Insbesondere kann der erste Sensor 115 in dem ersten Anschlusselement 211 oder dem zweiten Anschlusselement 221 angeordnet sein. Der erste Sensor 115 kann erfindungsgemäß als Durchflusssensor ausgebildet werden. Ist der Sensor 115 beispielsweise in dem ersten Anschlusselement 211 oder dem zweiten Anschlusselement 221 angeordnet, so kann der Sensor 115 als Durchflusssensor den Austausch bzw. den Massenstrom des Fluids zwischen dem ersten Polstermodul 110 und dem zweiten Polstermodul 220 messen. Basierend auf dem gemessenen Fluss des Massenstroms des Fluids kann auf eine Druckbelastung des ersten Polstermoduls 110 und des zweiten Polstermoduls 210 geschlossen werden.

Das erste Polstermodul 110 und das zweite Polstermodul 220 können beispielsweise wie das oben beschriebene Polstermodul 110, 117 ausgebildet werden. So kann das erste Polstermodul 110 und/oder das zweite Polstermodul 220 beispielsweise einen elastischen offenzelligen Schaumstoff mit fluiddurchlässigen Zellwänden aufweisen.
Das erste Anschlusselement 211 und das zweite Anschlusselement 221 sind in Fig. 2 zur besseren Darstellung vergrößert dargestellt. Im Allgemeinen können das erste Anschlusselement 211 und das zweite Anschlusselement 221 derart klein ausgebildet werden, dass ein Spalt zwischen dem ersten Polstermodul 110 und dem zweiten Polstermodul 220 klein ist bzw. dass kein Spalt zwischen dem ersten Polstermodul 110 und dem zweiten Polstermodul 220 vorliegt. Ferner weist das System 200 aus Fig. 2 ein drittes Polstermodul 230 auf, auf welches zumindest ein dritter Anteil des Körpergewichts der Person übertragbar ist. Das erste Polstermodul 100 weist ein weiteres erstes Anschlusselement 212 und das dritte Polstermodul 230 weist ein drittes Anschlusselement 231 auf. Das weitere erste Anschlusselement 212 und das dritte Anschlusselement 231 sind derart ausgebildet, dass das weitere erste Anschlusselement 212 lösbar an das dritte Anschlusselement 231 ankoppelbar ist, um das erste Polstermodul 110 lösbar mit dem dritten Polstermodul 130 zu befestigen.
Ferner ist wie in Fig. 1 dargestellt ein weiteres Polstermodul 117 an der Kopfstütze 102 angebracht. Ein weiterer Sensor 118 ist mit der Ausleseeinheit 221 gekoppelt.
Das zweite Polstermodul 220 oder dritte Polstermodul 230 weist z.B. ein zweites bzw. drittes fluiddichtes Innenvolumen auf, sodass bei Änderung einer Belastung des zweiten oder dritten Polstermoduls 220, 230 eine Änderung der physikalischen Größe des Fluides innerhalb des zweiten oder dritten Polstermoduls 220, 230 generierbar ist. In dem zweiten bzw. dritten fluiddichten Innenvolumen kann entsprechend ein zweites oder drittes Füllmaterial 222, 232 (z.B. aus einem offenzelligem Material) eingesetzt werden.
Das zweite Polstermodul 220 weist einen zweiten Sensor 223 und das dritte Polstermodul 230 weist einen dritten Sensor 233 zur Messung der Änderung der physikalischen Größe des Fluides auf. Wenn verschiedene Polstermodule 110, 117, 220, 230 oder alle Polstermodule 110, 117, 220, 230 jeweils zumindest einen Sensor 115, 118, 223, 233, welche in Inneren oder außerhalb der Polstermodule 110, 117, 220, 230 angeordnet sein können, aufweisen, kann ein Druckverteilungsmuster über die gesamte Fläche der entsprechenden Polstermodule 110, 117, 220, 230 gemessen werden und somit, wie oben beschrieben, z.B. eine bestimmte Liegeposition oder eine gewünschte andere vordefinierte Position der Person bestimmt werden.
Das zweite Anschlusselement 221 verbindet das zweite Innenvolumen selektiv mit einer Umgebung des zweiten Polstermoduls 221, wobei das erste Anschlusselement 211 und das zweite Anschlusselement 221 derart gekoppelt sind, dass ein Fluidaustausch zwischen dem ersten Innenvolumen und dem zweiten Innenvolumen bereitstellbar ist. Mittels der beispielhaften Ausführungsform kann das erste Anschlusselement 211 und das zweite Anschlusselement 221 eine fluiddichte Verbindung zwischen dem ersten Innenvolumen und dem zweiten Innenvolumen herstellen. Somit beeinflusst eine Änderung der physikalischen Größe des Fluides in dem ersten Polstermodul 110 eine Änderung der physikalischen Größe des Fluides in dem zweiten Polstermodul 220. Somit können mehrere Polstermodule 110, 117, 220, 230 miteinander befestigt werden, wobei es ausreicht zumindest einen Sensor 115 in einem der Polstermodule 110, 117, 220, 230 anzuordnen. In Fig. 2 weist alternativ dazu jedes der Polstermodule 110, 117, 220, 230 einen entsprechenden 115, 118, 223, 233 auf.

Die Anschlusselemente 211, 212, 221, 231 können beispielsweise jeweils ein Ventil aufweisen, welches erst einen fluiddurchlässigen Zustand einnimmt, wenn entsprechende Anschlusselemente 211, 212, 221, 231 miteinander verbunden sind.

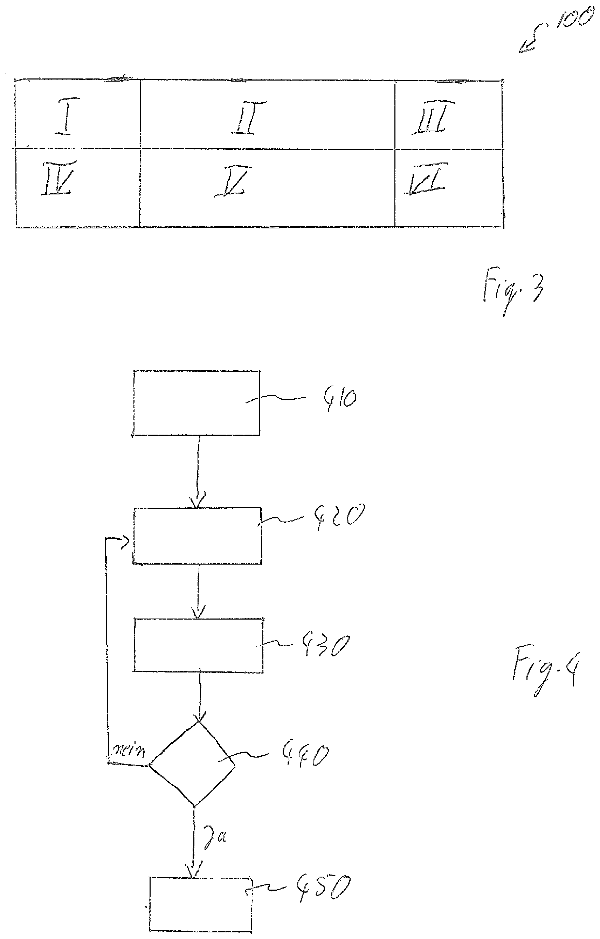

**Fig. 3** zeigt eine beispielhafte Darstellung des Systems 100 mit mehreren Polstermodulen I bis VI. In Fig. 3 bilden mehrere Polstermodule I bis VI beispielsweise eine Matratze. Die Polstermodule I bis VI sind entsprechend den Polstermodulen 110, 117, 220, 230 aus Fig. 2 ausgebildet und mittels entsprechender Anschlusselemente 211, 212, 221, 231 miteinander modular verbunden. Die Anschlusselemente 211, 212, 221, 231 sind zur besseren Übersicht nicht dargestellt.

Beispielsweise kann ein Polstermodul I bis VI eine Vielzahl von Anschlusselementen 211, 212, 221, 231 aufweisen. Beispielsweise kann ein Polstermodul I bis VI an gegenüberliegenden Seitenflächen oder an allen Seitenflächen entsprechende Anschlusselemente 211, 212, 221, 231 aufweisen. Somit kann eine beliebige Vielzahl von weiteren Polstermodulen I bis VI untereinander gekoppelt werden. Ferner können die Polstermodule I bis VI an beliebigen Stellen miteinander verbunden werden, so dass beliebige Muster von Polstermodulen I bis VI, z.B. eine beliebige Matratzenform und - größe gebildet werden können.

Mit der Anordnung mehrere Polstermodule I bis VI kann dadurch ein Druckverteilungsmuster gemessen werden und beispielsweise festgestellt werden, ob die Person flach mit seinem Rücken auf den Polstermodulen I bis VI auffliegt, seitlich bzw. in einer Seitenposition auf den Polstermodulen I bis VI auffliegt, oder ob die Person auf den Polstermodulen I bis VI sitzt. Dies ist möglich, da z.B. jede Liegeposition eine charakteristische Druckverteilung entlang des Polstermoduls I bis VI aufweist.
**Fig.4** zeigt einen möglichen Verfahrensablauf des erfindungsgemäßen Verfahrens zum Detektieren von Patientenbewegungen bzw. einer Bewegung einer Person insbesondere mit der Polstervorrichtung 100 aus Fig. 1 und dem System 200 aus Fig. 2.
In Verfahrensschritt 410 setzt oder legt sich der Patient auf z.B. eine Matratze bestehend aus zumindest einem oben beschriebenen Polstermodul 110. Im Anschluss daran kann in Verfahrensschritt 420 die Druckverteilung gemessen werden.
Durch den Druckanstieg kann beispielsweise das System 200 bzw. die Polstervorrichtung 100 aktiviert werden. Das System 200 ist dann sozusagen scharf geschaltet und ein Alarm kann ausgelöst werden.
In Verfahrensschritt 430 werden Abweichungen der Druckverteilung bzw. eine Änderung ( Massenstromänderung) der physikalischen Größe ( Massenstrom) des Fluides gemessen.
Anschließend wird in Verfahrensschritt 440 evaluiert, ob es sich um eine kritische Änderung der physikalischen Größe des Fluides, welche z.B. indikativ für ein Herausfallen des Patienten aus einem Bett ist, handelt. Liegt keine kritische Änderung der physikalischen Größe des Fluides vor, wird kein Alarm ausgelöst und es wird weiter das Polstermodul auf eine Änderung der physikalischen Größe des Fluides untersucht.

Falls es sich um eine kritische Änderung der physikalischen Größe des Fluides handelt, wird in Verfahrensschritt 450 ein Alarm ausgelöst und damit z.B. ein Pflegepersonal benachrichtigt.
Ergänzend ist darauf hinzuweisen, dass "umfassend" keine anderen Elemente oder Schritte ausschließt und "eine" oder "ein" keine Vielzahl ausschließt. Ferner sei darauf hingewiesen, dass Merkmale oder Schritte, die mit Verweis auf eines der obigen Ausführungsbeispiele beschrieben worden ist, auch in Kombination mit anderen Merkmalen oder Schritten anderer oben beschriebener Ausführungsbeispiele verwendet werden können. Bezugszeichen in den Ansprüchen sind nicht als Einschränkung anzusehen.

**Bezugszeichenliste:**

| | | | |
|---|---|---|---|
| 100 | Polstervorrichtung | 220 | zweites Polstermodul |
| 101 | Bett | 221 | zweites Anschlusselement |
| 102 | Kopfstütze | 222 | zweites Füllmaterial |
| 103 | Bettgestell | 223 | zweiter Sensor |
| 104 | Verbindungsschlauch | | |
| | | 230 | drittes Polstermodul |
| 110 | Polstermodul, erstes Polstermodul | 231 | drittes Anschlusselement |
| 111 | erste Kammer | 232 | drittes Füllmaterial |
| 112 | zweite Kammer | 233 | dritter Sensor |
| 113 | Füllmaterial | | |
| 114 | weiteres Füllmaterial | I-VI | Polstermodule |
| 115 | Sensor, erster Sensor | | |
| 116 | weiterer Sensor | 410 | Verfahrensschritt I |
| 117 | weiteres Polstermodul | 420 | Verfahrensschritt II |
| 118 | weiterer Sensor | 430 | Verfahrensschritt III |
| 119 | Koppelelement | 440 | Verfahrensschritt IV |
| | | 450 | Verfahrensschritt V |
| 120 | Steuereinheit | | |
| 121 | Ausleseeinheit | | |
| 122 | Sende-/Empfangseinheit | | |
| | | | |
| 130 | Fluidpumpe | | |
| 131 | Fluidverbindung | | |
| | | | |
| 200 | System | | |
| | | | |
| 211 | erstes Anschlusselement | | |
| 212 | weiteres erstes Anschlusselement | | |

## Patentansprüche

1. Polstervorrichtung (100) zum Überwachen von Bewegungen einer Person, wobei das Polstervorrichtung (100) aufweist
ein Polstermodul (110), auf welches zumindest ein Anteil eines Körpergewichts einer Person übertragbar ist,
wobei das Polstermodul (110) ein Innenvolumen aufweist, in welchem ein Füllmaterial (113) aus einem offenzelligen Schaumstoff angeordnet ist,
wobei der offenzellige Schaumstoff fluiddurchlässige Zellwände aufweist,
wobei das Füllmaterial (113) mit einer Umhüllung fluiddicht umhüllt ist, sodass bei Änderung einer Belastung des Polstermoduls (110) eine Änderung einer physikalischen Größe eines Fluides innerhalb des Polstermoduls (110) generierbar ist,
einen Sensor (115), und
ein Koppelelement (119), welches mit dem Innenvolumen gekoppelt ist, wobei an das Koppelelement (119) der Sensor (115) derart angekoppelt ist, dass mittels des Sensors (115) die Änderung der physikalischen Größe des Fluides in dem Innenvolumen messbar ist,
wobei der Sensor (115) ein Durchflusssensor ist und die physikalische Größe der Massenstrom des Fluides in das Innenvolumen hinein oder hinaus ist.

2. Polstervorrichtung (100) gemäß Anspruch 1, ferner aufweisend
den Sensor (115), welcher in dem Polstermodul (110) angeordnet ist und mittels des Koppelelements (119) an das Innenvolumen gekoppelt ist, und
wobei der Sensor (115) mit einer Ausleseeinheit (121) derart koppelbar ist, dass Signale, welche indikativ für die Änderung der physikalischen Größe sind, von der Ausleseeinheit (121) auslesbar sind.

3. Polstervorrichtung (100) gemäß Anspruch 1, ferner aufweisend
den Sensor (115), welcher außerhalb des Polstermoduls (110) angeordnet ist und mittels des Koppelelements (119) an das Innenvolumen gekoppelt ist, und
wobei der Sensor (115) mit einer Ausleseeinheit (121) derart koppelbar ist, dass Signale, welche indikativ für die Änderung der physikalischen Größe sind, von der Ausleseeinheit (121) auslesbar sind.

4. Polstervorrichtung (100) gemäß einem der Ansprüche 2 bis 3,
wobei der Sensor (115) ausgebildet ist, die Änderung der physikalischen Größe quantitativ zu messen.

5. Polstervorrichtung (100) gemäß einem der Ansprüche 2 bis 4, ferner aufweisend
ein weiteres Koppelelement (119), welches mit dem Innenvolumen gekoppelt ist,
wobei an das weitere Koppelelement (119) ein weiterer Sensor (116) derart ankoppelbar ist, dass mittels des weiteren Sensors (116) die Änderung der physikalischen Größe in dem Innenvolumen messbar ist, und
wobei das weitere Koppelelement (119) beabstandet von dem Koppelelement (119) angeordnet ist.

6. Polstervorrichtung (100) gemäß Anspruch 5,
wobei das Polstermodul (110) ferner einer erste Kammer (111) mit dem Füllmaterial (113) und eine zweite Kammer (112) mit einem weiteren Füllmaterial (114) aufweist,
wobei die erste Kammer (111) und die zweite Kammer (112) fluiddicht zueinander ausgebildet sind.

7. Polstervorrichtung (100) gemäß Anspruch 6,
wobei das Koppelelement (119) an die ersten Kammer (111) gekoppelt ist, und
wobei das weitere Koppelelement (119) an die zweite Kammer (112) gekoppelt ist.

8. Verfahren zum Überwachen von Bewegungen einer Person, wobei das Verfahren aufweist
Messen einer Änderung einer physikalischen Größe eines Fluides in einem Innenvolumen eines Polstermoduls (110) mittels eines Sensors (115), welcher an ein Koppelelement (119) gekoppelt ist,
wobei das Koppelelement (119) mit dem Innenvolumen gekoppelt ist,
wobei auf das Polstermodul (110) zumindest ein Anteil eines Körpergewichts einer Person übertragbar ist,
wobei in dem Polstermodul (110) ein Füllmaterial (113) aus einem offenzelligen Schaumstoff angeordnet ist,
wobei der offenzellige Schaumstoff fluiddurchlässige Zellwände aufweist,
wobei das Füllmaterial (113) mit einer Umhüllung fluiddicht umhüllt ist, sodass bei Änderung einer Belastung des Polstermoduls (110) die Änderung der physikalischen Größe innerhalb des Polstermoduls (110) generierbar ist,
wobei der Sensor (115) ein Durchflusssensor ist und die physikalische Größe der Massenstrom des Fluides in das Innenvolumen hinein oder hinaus ist.

## Claims

1. Cushion device (100) for monitoring movements of a person, wherein the cushion device (100) comprises
a cushion module (110) on which at least a part of a body weight of a person is transferable,
wherein the cushion module (110) comprises an inner volume in which a filling material (113) made of an open-celled foam material is arranged,
wherein the open-celled foam material comprises fluid-permeable cell walls,
wherein the filling material (113) is enveloped with an envelope in a fluid tight manner, such that, when a load of the cushion module (110) changes, a change of a physical quantity of a fluid within the cushion module (110) is generatable,
a sensor (115), and
a coupling element (119) which is coupled with the inner volume, wherein the sensor (115) is coupled to the coupling element (119) such that, by means of the sensor (115), the change of the physical quantity of the fluid in the inner volume is measurable,
wherein the sensor (115) is a flow sensor and the physical quantity is the mass flow of the fluid into or out of the inner volume.

2. Cushion device (100) according to claim 1, further comprising
the sensor (115) which is arranged in the cushion module (110) and which is coupled to the inner volume by the coupling element (119), and
wherein the sensor (115) is coupable with a reading unit (121) such that the signals which are indicative for the change of the physical quantity are readable by the reading unit (121).

3. Cushion device (100) according to claim 1, further comprising
the sensor (115) which is arranged outside of the cushion module (110) and which is coupled to the inner volume by the coupling element (119), and
wherein the sensor (115) is coupable with a reading unit (121) such that signals which are indicative for the change of the physical quantity are readable by the reading unit (121).

4. Cushion device (100) according to one of the claims 2 to 3,
wherein the sensor (115) is configured for quantitatively measuring the change of the physical quantity.

5. Cushion device (100) according to one of the claims 2 to 4, further comprising
a further coupling element (119) which is coupled with the inner volume,
wherein a further sensor (116) is coupable to the further coupling element (119) such that, by means of the further sensor (116), the change of the physical quantity in the inner volume is measurable, and
wherein the further coupling element (119) is arranged spaced apart from the coupling element (119).

6. Cushion device (100) according to claim 5,
wherein the cushion module (110) further comprises a first chamber (111) with the filling material (113) and a second chamber (112) with a further filling material (114),
wherein the first chamber (111) and the second chamber (112) are configured fluid tightly with respect to each other.

7. Cushion device (100) according to claim 6,
wherein the coupling element (119) is coupled to the first chamber (111), and
wherein the further coupling element (119) is coupled to the second chamber (112).

8. Method for monitoring movements of a person, wherein the method comprising
measuring a change of a physical quantity of a fluid in an inner volume of a cushion module (110) by means of a sensor (115) which is coupled to a coupling element (119),
wherein the coupling element (119) is coupled with the inner volume,
wherein at least a part of a body weight of a person is transferable to the cushion module (110),
wherein in the cushion module (110) a filling material (113) made of an open-celled foam material is arranged,
wherein the open-celled foam material comprises fluid-permeable cell walls,
wherein the filling material (113) is enveloped by an envelope in a fluid tight manner, such that, when a load of the cushion module (110) is changed, the change of the physical quantity in the cushion module (110) is generatable,
wherein the sensor (115) is a flow sensor and the physical quantity is the mass flow of the fluid into or out of the inner volume.

## Revendications

1. Dispositif de rembourrage (100) pour la surveillance des mouvements d'une personne, **caractérisé en ce que** le dispositif de rembourrage (100) présente
un module de rembourrage (110), sur lequel au moins une partie d'un poids corporel d'une personne est transmissible,
le module de rembourrage (110) présentant un volume intérieur, dans lequel un matériau de remplissage (113) en une mousse à cellules ouvertes est agencé,
la mousse à cellules ouvertes présentant des parois cellulaires perméables au fluide,
le matériau de remplissage (113) étant enveloppé de manière étanche au fluide avec une enveloppe de sorte qu'en cas de modification d'une sollicitation du module de rembourrage (110), une modification d'une grandeur physique d'un fluide puisse être générée à l'intérieur d'un module de rembourrage (110),
un capteur (115), et
un élément de couplage (119), lequel est couplé au volume intérieur,
le capteur (115) étant accouplé à l'élément de couplage (119) de telle manière que la modification de la grandeur physique du fluide dans le volume intérieur peut être mesurée au moyen du capteur (115),
le capteur (115) étant un débitmètre et la grandeur physique étant le flux massique du fluide entrant ou sortant du volume intérieur.

2. Dispositif de rembourrage (100) selon la revendication 1, présentant en outre
le capteur (115), lequel est agencé dans le module de rembourrage (110) et est couplé au volume intérieur au moyen de l'élément de couplage (119), et
**caractérisé en ce que** le capteur (115) peut être couplé à une unité de lecture (121) de telle manière que des signaux, lesquels indiquent la modification de la grandeur physique, sont lisibles par l'unité de lecture (121).

3. Dispositif de rembourrage (100) selon la revendication 1, présentant en outre
le capteur (115), lequel est agencé hors du module de rembourrage (110) et est couplé au volume intérieur au moyen de l'élément de couplage (119) et
**caractérisé en ce que** le capteur (115) peut être couplé à une unité de lecture (121) de telle manière que des signaux, lesquels indiquent la modification de la grandeur physique, sont lisibles par l'unité de lecture (121).

4. Dispositif de rembourrage (100) selon l'une quelconque des revendications 2 à 3,
**caractérisé en ce que** le capteur (115) est réalisé pour mesurer quantitativement la modification de la grandeur physique.

5. Dispositif de rembourrage (100) selon l'une quelconque des revendications 2 à 4, présentant en outre
un autre élément de couplage (119), lequel est couplé au volume intérieur,
**caractérisé en ce qu'**un autre capteur (116) peut être accouplé à l'autre élément de couplage (119) de telle manière que la modification de la grandeur physique peut être mesurée dans le volume intérieur au moyen de l'autre capteur (116), et
**en ce que** l'autre élément de couplage (119) est agencé à distance de l'élément de couplage (119).

6. Dispositif de rembourrage (100) selon la revendication 5,
**caractérisé en ce que** le module de rembourrage (110) présente en outre une première chambre (111) avec le matériau de remplissage (113) et une deuxième chambre (112) avec un autre matériau de remplissage (114),
la première chambre (111) et la deuxième chambre (112) étant réalisées l'une par rapport à l'autre de manière étanche au fluide.

7. Dispositif de rembourrage (100) selon la revendication 6,
**caractérisé en ce que** l'élément de couplage (119) est couplé à la première chambre (111), et
**en ce que** l'autre élément de couplage (119) est couplé à la deuxième chambre (112).

8. Procédé de surveillance des mouvements d'une personne, **caractérisé en ce que** le procédé présente
la mesure d'une modification d'une grandeur physique d'un fluide dans un volume intérieur d'un module de rembourrage (110) au moyen d'un capteur (115), lequel est couplé à un élément de couplage (119),
l'élément de couplage (119) étant couplé au volume intérieur,
au moins une partie d'un poids corporel d'une personne étant transmissible sur le module de rembourrage (110),
un matériau de remplissage (113) en une mousse à cellules ouvertes étant agencé dans le module de rembourrage (110),
la mousse à cellules ouvertes présentant des parois cellulaires perméables au fluide,
le matériau de remplissage (113) étant enveloppé de manière étanche au fluide avec une enveloppe de sorte qu'en cas de modification d'une sollicitation du module de rembourrage (110), la modification de la grandeur physique puisse être générée à l'intérieur du module de rembourrage (110),
le capteur (115) étant un débitmètre et la grandeur physique étant le flux massique du fluide entrant ou sortant du volume intérieur.
